# EUROPEAN PATENT APPLICATION

(11) **EP 3 782 816 A1**
(43) Date of publication of application: **24.02.2021**
(21) Application number: 19192661.7
(22) Date of filing: 20.08.2019
(51) Int. Cl.: B33Y 80/00, B25J 9/00, A61C 9/00, A61B 5/00

(54) **PERSONALIZED TONGUE-MACHINE-INTERFACE**

(71) Applicant: Universität Basel Vizerektorat Forschung, 4001 Basel (CH)
(72) Inventor: The designation of the inventor has not yet been filed
(74) Representative: Mertzlufft-Paufler, Cornelius

(57) **Abstract**

For exploiting novel use-cases, in particular sophisticated human-machine interaction, with an intraoral electronic tongue monitoring system (1) designed to be worn by a user on the upper or lower jaw and featuring a support sheet (2) bearing a number of intraoral sensors (17) arranged in an array (4) for recording tongue movement and/or tongue pressure, it is proposed that the system (1) comprises at least one extraoral sensor (6) located outside of the oral cavity (3) delimited by the teeth (19) when the system (1) is in place, in particular such that extraoral and/or intraoral and/or interlabial movements of the tongue and/or lip pressure can be recorded with the system (1) and/or such that the system (1) may be used as an input device controlled through tongue movement using a human-machine-interface provided by the system (1)

## Description

The invention concerns an intraoral electronic tongue monitoring system, comprising a support sheet designed to be attached to a human upper jaw (maxilla) or lower jaw (mandible) in an oral cavity and a sensor array arranged on the support sheet.

In addition, the invention concerns several practical use-cases of such a system as well as a process for fabricating the system.

Systems as introduced above are intended to be worn by a patient by placing the support sheet with the sensor array in the oral cavity, for exampling by attaching the support sheet to the palatum durum (or simply "palate"), i.e. to the upper jaw (maxilla). When the system is in place, the sensor array can then be used to record movements of the tongue inside the oral cavity, for example with respect to the palatum durum or relative to the teeth of the upper jaw. Moreover, it is also possible to record the pressure that the tongue exerts on the palatum durum.

Currently, such intraoral electronic tongue monitoring system have mainly attracted interest within the research community. However, several important applications, in particular in the field of logopedics can be recognized, because information on the position, orientation and pressure of the tongue inside the oral cavity is important for the study and correction of speech and language defects, disorders in communication and swallowing disorders.

Tongue monitoring systems investigated in research are, however, often plagued by insufficient performance of the sensor array and typically not pleasant to wear by a patient, in particular over longer times.

It is therefore an object of the present invention to provide a system which alleviates these deficiencies. It is a further object of the present invention to extend the scope of applications, in which such a system can be used.

In accordance with a first aspect of the present invention, a system is provided according to claim 1, which solves the afore-mentioned problems. In particular the invention proposes an intraoral electronic tongue monitoring system as introduced at the beginning, which, in addition, is characterized in that the sensor array comprises at least one intraoral sensor and at least one extraoral sensor.

The at least one extraoral sensor may be characterized in that it is/they are positioned outside of the oral cavity delimited by the upper and lower teeth, when the system is worn by a user on the lower or upper jaw. In other words, a sensor positioned in the vestibule of mouth, i.e. in the space between the lips or cheeks and teeth, may be considered an extra-oral sensor.

In particular, said at least one extraoral sensor may be an interlabial sensor and/or and extralabial sensor.

An interlabial sensor may be understood here as an extraoral sensor positioned in the clearance between the lips (interlabial space), when the system is worn by a user.

An extralabial sensor may be understood here as an extraoral sensor positioned outside of the cavum oris delimited by the lips.

To sum up, the system may be characterized in that the sensor array may comprise at least one intraoral sensor and at least one extraoral sensor, in particular at least one interlabial sensor and/or at least one extralabial sensor.

For example, the at least one extraoral sensor may be an interlabial sensor (i.e. placed in the interlabial space between the lips, when the system is worn by a user) and/or a sensor placed outside of the oral cavity and also outside of the interlabial space, when the system is worn by a user. Hence, the sensor array may comprise at least one intraoral sensor and at least one extraoral sensor and/or at least one interlabial sensor.

Moreover, extra-oral sensors of the system may be further characterized in that they may be located outside of the cavum oris delimited by the lips, when the system is worn by a user, for example on a lead of the support sheet protruding from the cavum oris, as will be explained in greater detail below.

Thus, the sensor array of the system may be customized such that both intraoral tongue movement / orientation / position / pressure can be measured as well as movement / orientation / position / pressure of the tongue in the extraoral space. For example, the sensor array may be customized to measure and/or record tongue movement / orientation / position / pressure in front of the teeth and/or in front of the lips and/or in the interlabial space (i.e. in the clearance between the lips).

Most preferably for high sensing resolution, the sensor array may include a multitude of intraoral sensors along with a multitude of extraoral sensors, in particular a multitude of interlabial sensors and/or a multitude of extralabial sensors.

By providing such additional sensing functionality, the range of applications in which such a system can be used is greatly expanded, as will become obvious from the description of several use-cases herein. In particular, the system proposed herein is highly interesting to orofacial myofunctional therapy. For example, using the system presented herein interactive exercises may be designed to disorganize and reshape patterns of oral habits to optimize oral and facial functions. Such therapy is important, because improper function of the lips and tongue can cause issues in speech, breathing, sleeping, growth, chewing, and swallowing, to name a few.

As indicated by the title, the intraoral electronic tongue monitoring system according to the invention can be individually customized to the oral cavity of a user (e.g. a patient whose tongue is to be monitored by the system). This may be done by matching a surface of the support sheet with the topology of the cavity of that user. Based on such a matched surface, the system may be reversibly and repeatedly attached to an upper or lower jaw of the user. In other words, the system can be individually designed to be worn on the specific lower or upper jaw of a user.

Different from systems that have been proposed so far, the invention suggests that the support sheet may be matched to a lower jaw such that it can be worn on that lower jaw, in spite of the fact, that the tongue is taking up a large portion of the lower part of the oral cavity. A position of the system on the lower jaw is particularly possible by using a support sheet with a C-shape, which will be explained in greater detail below.

The sensor array of the system may be used for spatially and/or temporally resolving tongue pressure and/or tongue movement / orientation / position, in particular, in the intraoral and in the extraoral space, most preferably in the interlabial and/or extralabial space.

The support sheet of the system provides structural stability and can be film-like, in particular flexible, or in the form of a relatively stiff plate, for example.

In accordance with a second aspect of the present invention, a system is provided according to claim 2, which solves the afore-mentioned problems. In particular the invention proposes an intraoral electronic tongue monitoring system as introduced at the beginning, which, in addition, is characterized in that the system comprises data acquisition electronics for acquiring a sensor signal form the sensor array and control electronics configured to generate control signals based on said acquired sensor signal, wherein the control signals are designed to control a human-machine-interface of the system.

In other words, the system according to the second aspect of the invention may be characterized in that it implements a human-machine-interface by being configured to acquire data on tongue movement / position / orientation / pressure using the sensor array and data acquisition electronics and to generate control signals based on these data through control electronics.

Using the human-machine-interface implemented by the system, a user may thus generate control signals by tongue movement and/or tongue pressure for controlling a software application, such as a video game, or an actuator or other device through the human-machine-interface.

For this purpose, it is sufficient if the tongue touches the sensor array to generate said sensor signal from the array of sensors. As will be outlined below, several sensor locations within the oral cavity as well as outside of the oral cavity can be used for allowing accurate control of the human-machine-interface through tongue movement and/or tongue position and/or tongue pressure, comparable to the control of a touch-pad by finger movement and/or finger pressure.

Comparable to a joystick as an input device controlled through movement of a pivotable stick, the intraoral electronic tongue monitoring system may be used as an input device controlled through tongue movement and/or tongue position and/or tongue pressure. The system can thus provide data and control signals to an information processing system such as a computer, a tablet, or a mobile electronic device.

In other words, the system discussed herein allows accurate user-machine-interaction by providing a novel way of interacting through tongue movement and/or tongue pressure. This functionality allows novel use-cases, which will be discussed below.

According to the invention, it is of particular advantage to combine the features of the systems described by claims 1 and 2, respectively. Moreover, any of the above detailed sub-features may be used in combination to achieve improved performance and benefit for the user of the system.

According to the invention, there exist further advantageous embodiments solving the aforementioned problems, which are described in the sub-claims and in the following. The features of these embodiments may be combined with any of the afore-mentioned features of the system to enhance the user benefit.

In the following, the term "intraoral" may refer to positions within the oral cavity, which is delimited by the teeth, i.e. by the posterior teeth (premolars and molars) and the anterior teeth (incisors and canines). Furthermore, the term "buccal" may refer to positions oriented towards a cheek, the term "labial" may refer to position oriented towards the lips, and the term "palatinal" may refer to positions oriented towards the palate.

For example, one sophisticated embodiment suggests that the system comprises at least one actuator for providing feedback perceptible to the tongue.

Such a design pushes the system to a second level of human-machine-interaction as it enables an information processing system, which may be part of the system, to address the user by the actuator and thus to send feedback to the user. In other words, bidirectional communication between the user and the information processing system can be achieved.

In detail, the user may send control signals to the information processing system using the afore-mentioned human-machine-interface, whereas the information processing system may send control or feedback signals designed for controlling said actuator to the tongue monitoring system (thus establishing a machine-human-interface), thereby providing feedback perceptible to the user and/or thereby communicating with the user through the actuator.

Preferably, the actuator may be an electromechanical actuator, for example in the form of a piezo-film or a DET-structure (as described below), for providing mechanical feedback, in particular in the form of mechanical vibrations.

In particular, the at least one actuator may be formed by at least one of the sensors of the array of sensors. Preferably, the system may comprise several of such actuators for providing feedback at different locations inside or outside of the oral cavity and/or to different portions of the tongue.

By providing haptic feedback to the user, using the actuator outlined above, the system is capable of achieving a new level of user-machine-interaction. Not only can control signals and data be inputted through a human-machine-interface of the system to an information processing system. The information processing system, which may be an external component or part of the tongue monitoring system presented herein, can also interact with the user by addressing the user through the actuator. This addressing can be done in a way that is perceptible even to people with handicaps, such as blindness or deafness, or people with temporarily limited awareness, e.g. in extreme situations, as the tongue is a highly sensitive sensory organ.

For accurate control of the actuator, the system may comprise an electronic driving unit configured to provide a drive signal to said at least one actuator. By this approach, interactive feedback may be provided to a user of the system.

In particular, the drive signal may be provided in response to a signal received from the sensor array.

The system may also comprise a, preferably wireless, communication module for bidirectional communication with an external electronic device. This communication module can additionally or alternatively enable the interactive feedback through interaction with the external electronic device.

According to a preferred embodiment, which is intended for application cases in which the system is to be worn over longer time, all electronic components of the system, in particular the communication module, the electronic driving unit, the data acquisition electronics and the control electronics can be arranged inside of the oral cavity, when the system is worn by a user. This is possible by using available highly miniaturized electronic components and attaching them to the support sheet.

According to another design, all electronic components of the system, except the array of sensors, may be located out of the oral cavity, in particular on a lead of the support sheet as described below. Such a design has the advantage that it poses fewer threats to a user, as components featuring an electrical voltage are located outside of the oral cavity.

Using a design featuring a communication module as described above, control signals for driving said at least one actuator or a feedback signal can be received by the system from the external electronic module and used for driving the at least one actuator. Such control signals or feedback signal may be received by the tongue monitoring system, in particular in response to a sensor signal detected with the sensor array and send via the communication module from the system to the external electronic module, which may be part of a tablet or other electronic device.

The communication module may be placed on an intraoral part of the system; preferably, however, it is to be placed on an extraoral portion of the system, in particular on a lead of the support sheet, which will be explained in detail below.

The communication module may be configured to send signals received from the sensor array to the external electronic device and/or to receive control signals for driving said at least one actuator from the external electronic device.

In particular, control signals for driving the at least one actuator of the system in response to a signal detected with the sensor array may be received wirelessly by the tongue monitoring system using the communication module.

To enhance the comfort of wearing the intraoral electronic tongue monitoring system, it is of great advantage if an intraoral portion of the system features a maximum material thickness of less than 2.0 mm, preferably of less than 1.5 mm, most preferably of less than 1.0 mm.

This maximum material thickness may be measured in particular with respect to a stack comprising the support sheet, a sensing film bearing the sensor array and a cover film.

Likewise of advantage for enhanced user comfort are designs, in which the support sheet is 3D-printed or thermoformed and/or wherein the support sheet shows a thickness of less than 1.0 mm, preferably of less than 0.5 mm, most preferable of less than 0.3 mm.

By such features, the system, in particular the support sheet, can be designed as an ultra-thin device which is not impeding the natural movement of the tongue in the oral cavity, when the system is worn by a user. This is of particular importance in logopaedic applications.

To enhance the measurement accuracy and performance when monitoring the tongue with the system as well as to enable ultra-thin designs of the system, one preferred embodiment suggests that the sensors of the sensor array are capacitive sensors. This is because capacitive sensors offer high measurement resolution and can be obtained by patterning thin films thus allowing very thin designs.

Even more preferably, each of the capacitive sensors may be formed by a dielectric elastomer transducer (DET), comprising a soft and reversibly compressible dielectric layer sandwiched between two conformable electrodes.

Using such DET-sensors, the at least one actuator described above may be formed by such a DET, since DETs can be used both as sensor and actuator devices.

The use of DETs offers manifold advantages, including high sensitivity, conformability of the sensors, possibility of achieving ultra-thin and low-power sensor designs as well as combined actuator/sensor-devices, and very high spatial and temporal resolution to name a few.

To achieve these advantages, the DET-sensors may have further features. For example, at least one of the two conformable electrodes may be applied on an elastomer and/or the dielectric layer may comprise a fluid. Such features enable a high conformity of the sensors as well as increased sensitivity. For the same reason, the dielectric layer can also be laterally displaceable.

According to a preferred design, at least one of said DETs may show an aspect ratio of thickness to width of less than 1:100, preferably less than 1:1000. Using this approach, an ultra-thin sensing film can be designed, as outlined below.

Accordingly, the dielectric layer of the DETs may have a submicron thickness and/or it may be able to reversibly take up a fluid, in particular a liquid. Moreover, the dielectric layer may contain nanoscale particles and/or comprise a network of polymer fibers embedded in a liquid. Such features are all of advantage for increasing the relative capacitance change and hence the sensitivity of the sensors.

To enhance the durability and robustness of the DET structures, it is of advantage if the electrodes are separated from said dielectric layer by a dielectric and elastically incompressible buffer layer such as PDMS.

With respect to the support sheet, another favorable design suggests that the support sheet includes a lead protruding from the oral cavity for supporting said at least one extraoral sensor, in particular said at least one interlabial sensor. This lead may have the form of a one-sided bridge.

Preferably, the lead may protrude from the oral cavity in the area of the incisors. Furthermore, it may show a width of less than the four upper incisors. Such designs are favorable because they safeguard that the system does not hinder the user to speak naturally when wearing the system in the mouth.

In addition, the lead may feature electrical connection lines routing sensor signals from intraoral sensors of the sensor array onto an extraoral area of the lead. In this extraoral area of the lead, an electromechanical interface, for example in the form of an electrical plug or electrical contact pads, may be formed. Using such an interface, sensor signals may be sent from the oral cavity to the outside via cables attached to the interface. Moreover, the lead may bear electronic components such as signal amplifiers and/or data acquisition electronics and/or a wireless communication module and/or a battery.

Concerning the layout of the sensor array, myriad configurations are possible.

In particular, the sensors of the sensor array, in particular the intraoral sensors, may be distributed over a lower surface of the support sheet facing the tongue, when the system is designed to be attached to the upper jaw or to an upper surface of the support sheet facing the tongue, when the system is designed to be attached to a lower jaw.

Preferably, several of the intraoral sensors may by placed within a palatinal area enclosed by teeth of the upper or lower jaw.

The interlabial sensors of the sensor array are useful for measuring lip pressure, when upper and lower lip are pressed onto each other, which is important in particular for logopaedic applications and anti-snoring therapies.

According to another particular embodiment, at least one extraoral sensor may be placed on the lead of the support sheet described previously.

Moreover, at least one extraoral sensor of the sensor array may be placed such that it can be reached by the tip of the tongue, when the system is attached to the upper or lower jaw in the oral cavity. Such a design is highly useful for tongue skills training, as will be explained in greater detail below.

In addition, the extraoral sensors may be placed outside of the clearance between the lips and/or out of reach of the lips, when the system is attached to the upper or lower jaw. Such a design is highly useful for fine skills training of the tongue muscle and/or training of the tongue muscle tone, which can by highly beneficial, in particular for treating snoring or sleep apnoe.

According to another preferred embodiment, the sensor array may comprise at least one extraoral sensor facing a buccal or labial face of a tooth of the upper or lower jaw. Preferably, the extraoral sensors may be placed at the location of a canine teeth and/or of at least one posterior teeth, preferably a pre-molar.

According to another design, the sensor array may comprise at least one extraoral sensor facing a labial face of an anterior tooth.

Moreover, the sensor array may comprise at least one intraoral sensor facing a palatinal face of a tooth of the upper or lower jaw, preferably of a canine teeth or a posterior teeth, preferably a pre-molar.

According to another design, the sensor array may comprise at least one intraoral sensor facing a palatinal face of an anterior tooth.

In other words, the system may comprise sensors oriented towards the outer sides of teeth of the upper or lower jaw and/or sensors oriented towards the inner palatinal sides of teeth of the upper or lower jaw.

Such sensor locations can be reached by the tongue, as the tongue is highly movable within the oral cavity and can be accurately steered by a user, in particular after training. Moreover, such sensor locations are highly suitable for generating the sensor signal used for controlling the human-machine-interface.

Such sensor layouts are thus innovative in that they can be designed for recording tongue movement along buccal or facial sides of teeth of the upper and lower jaw. By recording such movements, more information can be gathered as compared to designs which feature only sensors oriented towards inner sides of the teeth. In addition, such tongue movements are particularly suited as input movements for generating control signals for control of the human-machine-interface of the system explained above. Hence, more accurate and controllable input via the human-machine-interface can be achieved with such designs.

If the system with such a sensor design is attached to the upper or lower jaw in the oral cavity, a pressure exerted by the tongue on the palatinal and/or buccal/labial face of the teeth can be measured/detected, respectively. In particular the buccal or labial facing intraoral sensors appear highly useful to be used as a human-machine-interface to be touched / controlled by the tongue.

In addition, with such designs the tongue pressure can be measured more accurately, which the tongue exerts on the teeth. Using palatinal, buccal or labial facing intraoral sensors is also of advantage for accurately determining the position and/or orientation and/or movement of the tongue, within the oral cavity, respectively. Such measurements are of particular importance for applications such as speech training or treatment of snoring or sleep apnea and can all be derived from spatially and/or temporally resolved pressure data, acquired with the array of sensors. In particular, the determination of the exact position of the tongue is of vital importance for a myofunctional tongue therapy to treat snoring and other malfunctions.

According to another preferred embodiment, the sensor array may comprise one central sensor patch located at the so-called tongue resting position, which is the natural position at which the tongue is at rest within the oral cavity.

This design can be further improved by providing two buccal sensor patches facing left and right buccal side surfaces of teeth of the upper or lower jaw, preferably at the location of canine teeth and/or posterior teeth.

In an even further improved design in terms of device functionality, the system may feature additionally or alternatively two palatinal sensor patches facing left and right palatinal side surfaces of teeth of the upper or lower jaw, preferably of posterior teeth.

Using this approach, a design is proposed that is dedicated for accurately resolving 3d-movements of the tongue within the oral cavity which is of interest for numerous applications.

Such recording is particularly improved in case each of said sensor patches features at least two, preferably at least three, separate sensors for spatially and/or temporally resolving tongue pressure as exerted on the respective sensor patch.

The sensor array may be applied as a flexible sensing film to a surface of said support sheet, for example by gluing or deepdrawing or thermoforming. This approach is of advantage because the relative positions of the sensors within the array can be pre-defined on the sensing film, prior to attachment to the support sheet. Moreover, the sensing film can be produced as a mass product to be attached to support sheets individually customized to the need of individual users.

The sensing film may be very thin. For example, it may show a thickness of less than 500 µm, preferably less than 100 µm, and most preferably less than 50 µm. Moreover, it may be stretchable. In the latter case, it is of particular advantage of using stretchable DETs as sensors.

The support sheet may cover both the palatum durum at least partly and several teeth of the upper or lower jaw, in particular at least two anterior teeth on each side. In addition, the support sheet may cover individual buccal and palatinal faces of teeth of the upper or lower jaw, preferably on both sides.

According to a preferred embodiment which is suitable for wearing the system on the lower jaw and also for improved comfort, when wearing the system on the upper jaw, the system, in particular the support sheet and the sensing film, may show a C-shape with a central recess or gap. This recess may leave a central and rear part of the palate free for natural tongue contact, when the system is worn by a user on the upper jaw or provide enough space for the tongue, when the system is worn on the lower jaw. Such a design is also beneficial for avoiding the pharyngeal reflex (gagging reaction), because the recess can be such that the support sheet does not touch the back of the tongue, the area around the tonsils, the uvula, and the back of the throat, when the system is worn by a user. This is of particular importance for persons with a hypersensitive gag reflex.

To achieve a more natural tongue feeling, when a user is wearing the system on his upper or lower jaw the surface of the support sheet facing the tongue may feature a reproduction of a topology of the upper or lower jaw in the oral cavity, in particular of the palatum durum (palate) and of teeth of the upper or lower jaw.

Preferably the sensing film, which may show a uniform thickness, closely follows said topology such that the topology is reproduced on the side of said sensing film facing the tongue.

The topology on the side of the support sheet in touch with the teeth may thus be repeated as a negative on the side of the support sheet, facing the tongue.

Preferably, the topology on the side of the support sheet facing the teeth may be a negative of a reproduction of a specific topology of an oral cavity of an individual patient, as will be explained in greater detail with respect to the fabrication process of the system.

In accordance with the present invention, there are also provided several novel use-cases of an intraoral electronic tongue monitoring system, which extend the scope of possible applications.

In particular there is provided a use of an intraoral electronic tongue monitoring system for measuring a pressure exerted by the tongue on single teeth of the upper or lower jaw, in particular on premolar and/or molar teeth. Such a method requires that the system offers a spatial sensor resolution in the order of the size of a single tooth.

A further use-case proposed by the invention is using an intraoral electronic tongue monitoring system for measuring a pressure distribution exerted by the tongue on a support sheet of the system and/or on a sensing film of the system.

Yet another use-case proposed by the invention is using an intraoral electronic tongue monitoring system for measuring interlabial pressure and/or for detecting extraoral movements of the tongue, in particular for tongue training.

According to the second aspect of the invention, there is also provided a use case in which an intraoral electronic tongue monitoring system is used as a human-machine-interface. As explained previously, through this human-machine-interface, a user of the system may interact with an information processing system such as a computer, a tablet, or a mobile electronic device. Moreover, he may generate control signals by tongue movement and/or tongue pressure for controlling a software application, such as a video game, or an actuator through the human-machine-interface. This use-case is thus particularly adapt for controlling a software application, for example a video game, or an actuator through tongue movement, respectively.

Preferably, a tongue pressure distribution may be spatially and/or temporally resolved by the system and read out for controlling said software / said actuator.

Most preferably, feedback perceptible to the tongue may be provided to a user by at least one actuator of the system.

These approaches may be useful for example for combat pilots in extreme situations, where loss of control of other parts of the body as the tongue has occurred.

In yet another use-case according to the invention, it is proposed to use an intraoral electronic tongue monitoring system as a training device for training of tongue movement and position and/or tongue muscle tonus. In this use case, it is of advantage if the system comprises at least one actuator for providing feedback perceptible to the tongue, as this allows more accurate training based on a feedback-loop.

For example, the actuator may provide electrical feedback, for example by an electrical current, and/or mechanical feedback, for example in the form of vibrations.

An intraoral electronic tongue monitoring system, in particular as detailed herein, may be used, for example, for rehabilitation exercises for persons having articulation impediments or dysphagia or neurologic disorders, to name a few.

All of the above use-cases, whose features may also be applied in combination, can most advantageously be performed with an intraoral electronic tongue monitoring system having features as explained previously herein and/or according to one of the claims directed towards such a system. In other words, features of the claims directed towards such a system can be combined with features of claims directed towards special uses of such a system.

Finally, another use-case according to the invention is to use a device as outlined herein for treating snoring or sleep apnea or speech impediments. For this purpose the sensing functionality can be used for detecting critical tongue movements, e.g. during sleep, and a possible actuator functionality can be used for providing feedback to the user to avoid critical situations such as snoring or sleep apnea.

To solve the afore-mentioned problem, a process for fabricating an intraoral electronic tongue monitoring system comprising a support sheet to be attached to a human upper or lower jaw in an oral cavity and a sensor array arranged on the support sheet, in particular as described previously and/or as defined by one of the claims directed towards such a system, is provided. This process is characterized in that sensors of the sensor array are, at least partly, preferably fully, fabricated on a separate film to form a sensing film and in that said sensing film is attached to said support sheet.

For fabricating the sensors, in particular DETs, on the film, standard micro-fabrication processes may be used, in particular batch processes.

The film forming the sensing film may preferably feature a thickness of less than 500 µm, most preferably of less than 100 µm.

Moreover, the film may be stretchable. Consequently, according to a specific variant of the process, the film as well as the sensors formed on the film are stretched during application of the sensing film to the support sheet.

In other words, the sensors used for the system may be stretchable.

For further protection of the sensor array, a cover film may be attached afterwards, preferably by thermo-forming, to a stack comprising the support sheet and attached sensing film.

According to a preferred embodiment, the support sheet may feature a lead protruding from the oral cavity (when the system is attached to the upper or lower jaw of the patient), as explained previously. In this case, it is preferable if the sensor film, in particular and the cover film, covers the lead. By such a design, sensors, in particular intra-labial and/or extraoral sensors, may be placed on the lead.

The above fabrication process can be further characterized. For example, according to the invention, the support sheet may be fabricated by casting or 3d-printing a 3D-model of an upper or lower jaw of an individual patient and by subsequent 3d-forming, in particular deepdrawing or thermo-forming, of the support sheet using said 3D-model.

In such a case, it is preferable, if said model includes a foundation for replicating a lead designed for bearing extraoral and/or interlabial sensors of the system.

As an alternative fabrication variant, the invention further suggest that the support sheet may be fabricated by acquiring 3D-data of a topology of an upper or lower jaw of an individual patient and by subsequent 3D-printing of the support sheet (2) as a negative of said topology, based on said 3D-data. As a result, the support sheet will thus accurately match said topology.

The acquisition of the 3D-data can be performed in particular by using an intraoral optical 3D-scanner.

Typically, the foundation used for replicating the lead is not part of the 3D-model obtained from the upper or lower jaw of the patient. Likewise, when 3D-printing the support sheet, a foundation not part of the topology of the upper or lower jaw may be formed.

According to a particular embodiment, after attaching the sensing film to the support sheet to form a stack, this stack may be formed, in particular by deep drawing or thermoforming, to match the shape of an oral cavity of an individual patient. Using this particular approach, the relative position of the sensor array on the support sheet can be accurately defined, because the support sheet may be still flat when the sensing film is attached onto it. At the same time, the support sheet can feature an upper surface which is a negative of the oral cavity in the area of the palatum durum and inner side surfaces of the upper teeth, by 3D-forming the complete stack of support sheet and attached sensing film afterwards.

Preferred embodiments of the present invention shall now be described in more detail, although the present invention is not limited to these embodiments: for those skilled in the art it is obvious that further embodiments of the present invention may be obtained by combining features of one or more of the patent claims with each other and/or with one or more features of an embodiment described or illustrated herein.

With reference to the accompanying drawings, where features with corresponding technical function are referenced with same numerals even when these features differ in shape or design:
- Fig. 1: shows an upper jaw of an individual patient,
- Fig. 2: shows a 3D-model of the jaw of figure 1 with an additional foundation,
- Fig. 3: illustrates a support sheet of a system according to the invention obtained by thermoforming using the 3D-model of figure 2,
- Fig. 4: illustrates the attachment of a sensing film onto the support sheet of figure 3 in a perspective view,
- Fig. 5: illustrates in a top view the locations of single sensor patches of the sensing film of figure 4 on the support sheet of figure 3,
- Fig. 6: illustrates the attachment of a cover film onto the stack of support sheet and attached sensing film shown in figure 4,
- Fig. 7: shows the resulting stack of support sheet and sensing film with attached cover film and
- Fig. 8: shows the final intraoral electronic tongue monitoring system after a central recess has been cut out from the support sheet, sensing film and cover film to result in a C-shaped device.

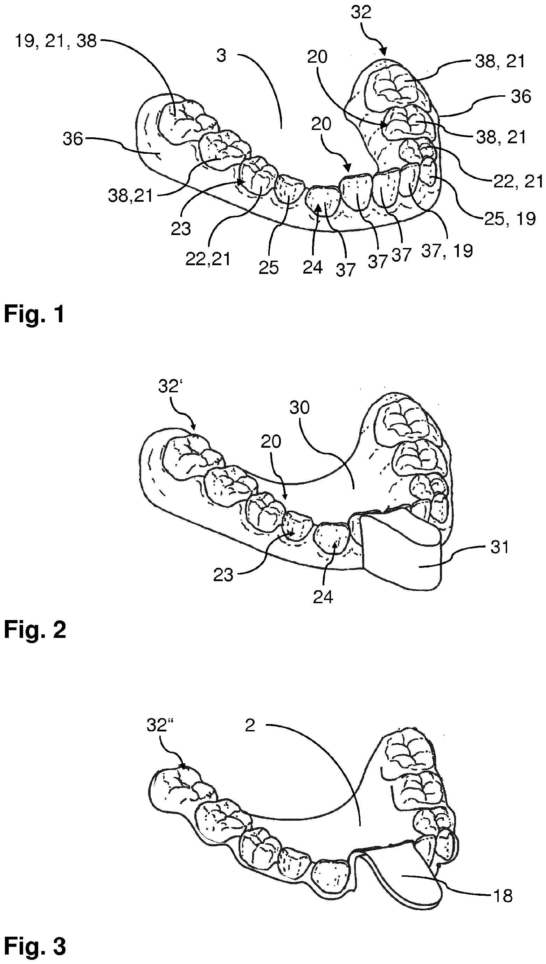

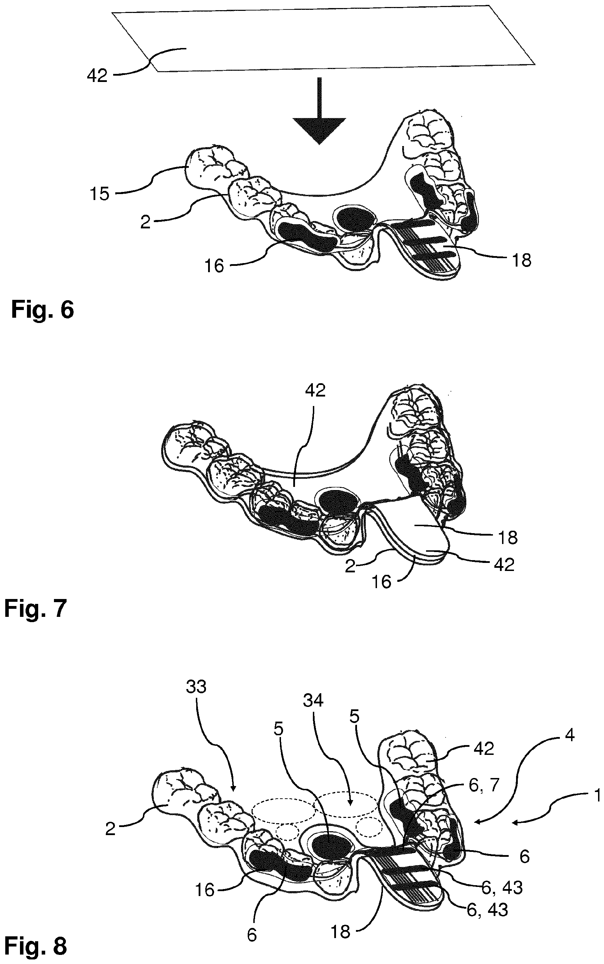

Figure 8 shows an intraoral electronic tongue monitoring system 1 according to the invention, comprising a support sheet 2 to be attached to the human upper jaw 36 visible in figure 1. The system 1 is intended to be worn by a user inside of the oral cavity 3 delimited by the teeth 19 of the jaw 36 (cf. figure 1). In other words, the lower side of the support sheet 2 visible in figures 8 and 3 is customized individually to the topology 32 of the upper jaw 36 of the user to allow reversible and repeated attachment of the support sheet 2 to the teeth 36 of the upper jaw 36. When the system 1 shown in figure 8 is in place, i.e. attached to the upper jaw 36, the upper side visible in figure 8 is directed towards the lower jaw and the tongue.

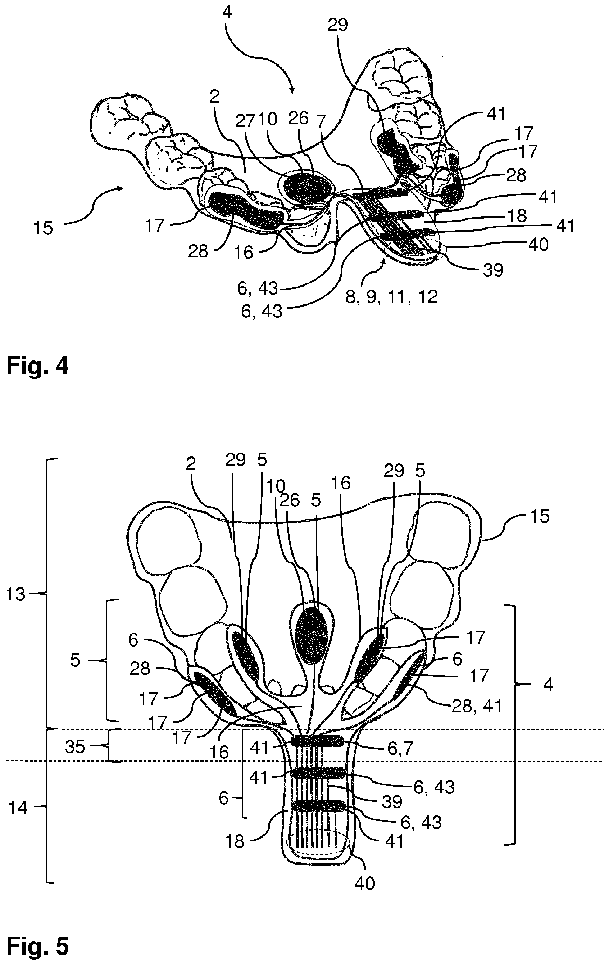

As is visible in figures 4 and 8, a sensor array 4 consisting of a multitude of sensors 17 is arranged on the support sheet 2 for recording movements of the tongue as well as pressure exerted by the tongue onto the support sheet 2, when the system 1 is in place on the upper jaw 36. As indicated in figure 8, the system 1 offers both intraoral sensors 5 as well as extraoral sensors 6 for recording tongue movement and/or pressure, i.e. tongue movement can also be detected outside of the oral cavity 3 delimited by the teeth 19.

The construction of the system 1 is best understood by understanding the fabrication process first:
To start with the physiology, figure 1 illustrates the position of single teeth 19 on an upper jaw 36 of a user of the system 1. Visible are incisors 37, canine teeth 25, pre-molars 22 as well as molar teeth 38. The C-row of teeth 19 thus delimits the oral cavity 3, in which the tongue is located, with a natural tongue resting position 27 on the upper jaw 36.

Indicated in figure 1 is also that the teeth 19 have outer surfaces, namely buccal faces 23 (cf. the molar teeth 38 and the pre-molars 22) facing the cheeks, and labial faces 24 (cf. the incisors 37 and canines 25) facing the lips. On the inner sides of the teeth 19, palatinal faces 20 of the teeth 19 are oriented towards the palate and the tongue.

As a first step in the fabrication of the system 1, 3D-data of the topology 32 of the upper jaw 36 of the user shown in figure 1 are acquired using an intraoral optical 3D-scanner. Based on these data, a 3D-model 30 resembling closely the upper jaw 36 is obtained by 3D-printing. Alternatively, such a 3D-model 30 can also be obtained by a casting process from the upper jaw 36 shown in figure 1.

As is visible in figure 2, the 3D-model 30 includes a foundation 31 for replicating a lead 18 designed for bearing the extralabial sensors 43 of the system 1, as is visible in figure 8.

As a next step, a support sheet 2 out of a thermoplastic material is thermo-formed from the 3D-model 30 by deepdrawing. As a result, the support sheet 2 shown in figure 3 is obtained featuring, the desired lead 18.

As the support sheet 2 is very thin, its upper surface (with respect to figure 3), facing the tongue when the system 1 is in place on the upper jaw 36, offers a topology 32", which closely resembles the topology 32' of the 3D-model 30, which itself is an accurate copy of the physiologic topology 32 of the upper jaw 36 of the user.

Alternatively, the support sheet 2 may be directly obtained from the 3D-data of the upper jaw 36 recorded with the intraoral optical 3D-scanner by 3D-printing the support sheet 2 as a negative of the topology 32, based on said 3D-data. In this case, a similar accurate copy of the topology 32 can be obtained on the support sheet 2.

In another step, the multitude of sensors 17 are micro-fabricated as a sensor array 4 by batch processes, using standard lithography tools, on an elastic and stretchable thin film. As a result, an elastic and stretchable sensing film 16 is obtained comprising the thin film and the sensor array 4.

The sensing film 16 is next cut into the five-finger-shape visible in figures 4 and 5, with a protruding portion matching the shape of the lead 18 of the support sheet 2.

The pre-cut sensing film 16 is next attached onto the thermo-formed support sheet 2 using a thin glue layer. The sensing film 16 is attached on the side of the support sheet 2 facing the tongue when the system 1 is in place. During this process, the sensing film 16 may be stretched to conformally adapt to the complex topology 32" of the support sheet 2. The result is visible in figure 5, illustrating the relative position of the sensing film 16 on the support sheet 2. Importantly, the sensing film 16 extends onto an extraoral portion 14 of the support sheet 2, in particular onto the lead 18 (cf. figures 5 and 6) .

As an optional step, illustrated in figures 6 and 7, a cover film 42 is next attached onto the stack 15 comprising the support sheet 2 with attached sensing film 16 for protection of the delicate sensors 17. The resulting structure consisting of a stack of support sheet 2, sensing film 16, and attached cover film 42 is illustrated in figure 7. The cover film 42 also covers the lead 18.

As a final step, a recess 34, indicated by dashed lines in figure 8, is cut out of the aforementioned stack. In conclusion, the final system 1 shown in figure 8, in particular its support sheet 2, shows a C-shape resembling the C-arc of the teeth 29 visible in figure 1. Due to the recess 34, a central and rear part of the palate is left free for natural tongue contact, when the system 1 is worn by a user on the upper jaw. In applications, where the system 1 is designed to be worn on the lower jaw by a user, the recess 34 may be designed such that enough free space is left for natural movement of the tongue.

Referring now to figure 5, displaying a top view on the stack 15 consisting of the support sheet 2 and attached sensing film 16 bearing the sensor array 4, the two dashed lines indicate the interlabial space which is delimited by the lips. The intraoral space (i.e. the oral cavity 3) is located above the upper of the two dashed lines, and the extra-oral space as well as the extraoral portion 14 of the system 1 is located below the upper of the two dashed lines, in particular below the lower of the two dashed lines.

The intraoral portion 13 of the system 1 shows a maximum material thickness with respect to the stack consisting of support sheet 2, sensing film 16 and cover film 42 of less than 1 mm and can thus be worn comfortably by a user over longer times. The support sheet 2 shows a thickness of less than 0.5 mm, and the sensing film 16 and cover film 42 of less than 0.1 mm, respectively.

As is visible in figure 5, the sensor array 4 comprises several intraoral sensors 5, which are positioned in the oral cavity 3 delimited by the teeth (cf. figure 1), when the system 1 is worn by the user. Moreover, the sensor array 4 comprises a multitude of extraoral sensors 6, namely
- several extraoral sensors 6 positioned on outer surfaces of the teeth 19 (cf. the buccal sensor patches 28),
- several interlabial sensors 7 positioned in the interlabial space delimited by upper a lower lib, and
- several extralabial sensors 43 positioned on the lead 18 and hence outside of the oral cavity 3 and outside of the cavum oris delimited by the lips.

In greater detail, sensor array 4 features a central sensor patch 26 positioned centrally on the palatum durum. With this patch, movement of the tongue to the tongue resting position 27 (cf. figure 4) can be detected.

In addition, sensor array 4 offers two palatinal sensor patches 29, detecting movement of the tongue along inner/palatinal side surfaces of the teeth 19, in particular on palatinal faces of the canine teeth 25 and the first premolars 22.

Sensor array 4 features also two buccal sensor patches 28 facing left and right buccal side surfaces of the canine teeth 25 and first pre-molar teeth 22 (cf. figure 1 and figure 5) and thus reaching out of the oral cavity into the extraoral space. With these patches 28, movement of the tongue in the extraoral space can be detected, in particular along the outer sides of the teeth 19 of the upper jaw 36, i.e. in the vestibule of mouth (the space between the lips or cheeks and teeth).

Of course, other sensor positions within the intraoral or extraoral space can be realized. For example, one design according to the invention suggests to use sensors facing palatinal faces of anterior teeth and/or labial faces of anterior teeth.

In addition to the extraoral sensors 6 of the two 28 buccal sensor patches 28, the system 1 also offers three additional extraoral sensor patches 41, namely one patch featuring several interlabial sensors 7 positioned in the interlabial space 35, and two patches each featuring extralabial sensors 43, located outside of the cavum oris delimited by the lips. The latter three patches are all located on the lead 18, as is visible in figures 4 and 5.

Each of the eight patches 26, 29, 28, 41 bears a minimum of three separate sensors 17 for spatially resolving tongue pressure on each of the patches and/or tongue movement over each patch.

Each of these sensors 17 is formed as a capacitive sensor, namely as a dielectric elastomer transducer (DET), and offers a very high sensitivity for spatially and temporally resolving tongue pressure exerted on the respective sensor patch 26, 29, 28, 41.

As indicated by the dashed line in figure 4, the system 1 also features an electromechanical interface 40 in the form of a socket, which can be electrically connected to a cable for measuring the sensor signal of the sensor array 4. The electromechanical interface 40 is connected to electrical connection lines 39 on the lead 18, which route sensor signals from the sensor array 4 to the electromechanical interface 40.

The central sensor patch 26 is equipped with an electromechanical actuator 10 for providing haptic feedback to the user in the form of vibrations, which are perceptible with the tongue. The actuator 10 is actually realized by one of the DET-sensors 17 of the central patch 26, as DETs can be used both as sensors and actuators.

As indicated in figure 4, the system 1 further comprises data acquisition electronics 8 for acquiring a sensor signal from the sensor array 4, control electronics 9 for generating control signals based on the acquired sensor signal, an electronic driving unit 11 for driving the actuator 10, and a communication module 12 for wireless communication with an external electronic device in the form of a tablet. These electronic components are all realized as miniaturized and low-weight ICs and attached to the lower side of the lead 18 (not visible in the figures) and thus placed outside of the oral cavity 3, when the system 1 is attached to the upper jaw 36.

The control signals are designed to control a human-machine-interface of the system 1. In other words, a user may generate said sensor signal by tongue movement and/or tongue pressure and thereby control signals for controlling a software application running on the tablet though the human-machine-interface. For this purpose, the system 1 spatially and temporally resolves tongue pressure distributions on the various sensor patches 26, 28, 29, 41 and provides a corresponding sensor signal. The pressure distributions are then read out by the system 1 for generating the control signals designed for controlling the software application on the tablet. These operations are performed by the control electronics 9, which feature also signal amplification circuitry, AD- and DA-converter, as well as logical circuits providing some intelligence to the system 1.

The tablet on the other hand can communicate wirelessly via the communication module 12 with the system 1 and send a feedback signal to the system 1. This feedback signal is then used to generate a driving signal using the electronic driving unit 11 to generate a haptic feedback to the user through the actuator 10.

By this approach, sophisticated user-machine-interaction - in both directions - can be accomplished, i.e. the user can control the software application on the table via his tongue and he can also sense feedback sent from the tablet to the system using his tongue.

Based on this approach, it is possible for example to train the muscle of the tongue by letting the user perform a video game on the tablet using the system 1 as an input device for generating control signals to be send wirelessly via the communication module from the system 1 to the tablet. For this purpose, it is sufficient if the tongue touches the sensor array 4 to generate a sensor signal.

As the sensor array 4 offers various sensors at different positions, in particular in the interlabial and extraoral space, various movements of the tongue can be differentiated. For example, it is possible to detect when the user is striking with this tongue along the outer surfaces of his teeth 19 and thus over the buccal sensor patches 28.

The extralabial sensors 43 on the lead 18 offer the possibility of training specific tongue movements, in which the user is touching the extraoral sensor patches 41 on the lead 18 with the tip of his tongue. Moreover, movements of the tongue in the extralabial space, i.e. outside of the cavum oris delimited by the lips, can be detected with the extralabial sensors 43.

In conclusion, the system 1 can be used as a training device for training of tongue movement and position and/or tongue muscle tonus. This functionality is highly attractive for treatment of snoring, sleep apnea or speech impediments.

Extraoral movements of the tongue in the vestibule of mouth, i.e. in the space between the lips or cheeks and teeth 19, can be detected with the buccal sensor patches 28 of the system 1.

Using the interlabial sensors 7, the pressure exerted by the lips can be resolved, in addition to the tongue pressure recorded by the other intraoral sensors 5 and extraoral sensors 6 of the sensor array 4. Such measurements are particularly interesting for logopedic tests.

As another use, the sensor array 4 can be used to measure the pressure that the tongue exerts on single teeth, for example on the pre-molars 22 using the buccal and palatinal sensor patches 28 and 29.

In summary, the invention aims at exploiting novel use-cases, in particular sophisticated human-machine interaction, with an intraoral electronic tongue monitoring system 1 designed to be worn by a user on the upper or lower jaw and featuring a support sheet 2 bearing a number of intraoral sensors 17 arranged in an array 4 for recording tongue movement and/or tongue pressure. To achieve this goal, it is proposed that the system 1 comprises at least one extraoral sensor 6 located outside of the oral cavity 3 delimited by the teeth 19 when the system 1 is in place, in particular such that extraoral and/or intraoral and/or interlabial movements of the tongue and/or lip pressure can be recorded with the system 1. Moreover, it is proposed to use the system 1 as an input device controlled through tongue movement using a human-machine-interface provided by the system 1.

### List of reference numerals

- 1: intraoral electronic tongue monitoring system
- 2: support sheet
- 3: oral cavity
- 4: sensor array
- 5: intraoral sensor
- 6: extraoral sensor
- 7: interlabial sensor
- 8: data acquisition electronics
- 9: control electronics
- 10: actuator
- 11: electronic driving unit
- 12: communication module
- 13: intraoral portion (of 1)
- 14: extraoral portion (of 1)
- 15: stack (of 2 and 16)
- 16: sensing film
- 17: sensor (of 4)
- 18: lead
- 19: tooth
- 20: palatinal face (of 19)
- 21: posterior tooth
- 22: pre-molar
- 23: buccal face
- 24: labial face
- 25: canine tooth
- 26: central sensor patch
- 27: tongue resting position
- 28: buccal sensor patch
- 29: palatinal sensor patch
- 30: 3d-model (of upper / lower jaw)
- 31: foundation (for replication of 18)
- 32: topology (of upper / lower jaw)
- 33: C-shape
- 34: recess
- 35: interlabial space
- 36: jaw
- 37: incisor
- 38: molar tooth
- 39: electrical connection line
- 40: electromechanical interface
- 41: extraoral sensor patch
- 42: cover film
- 43: extralabial sensor

## Claims

1. **Intraoral electronic tongue monitoring system** (1), comprising a support sheet (2) to be attached to a human upper or lower jaw in an oral cavity (3) and an sensor array (4) arranged on the support sheet (2), **characterized in that** the sensor array (4) comprises at least one intraoral sensor (5) and at least one extraoral sensor (6), in particular at least one interlabial sensor (7).

2. **Intraoral electronic tongue monitoring system** (1), in particular according to claim 1, comprising a support sheet (2) to be attached to an upper or lower jaw in an oral cavity (3) and an sensor array(4) arranged on the support sheet (2), **characterized in that**
- the system (1) comprises data acquisition electronics (8) for acquiring a sensor signal form the sensor array(4) and
- control electronics (9) configured to generate control signals based on said acquired sensor signal, wherein
- the control signals are designed to control a human-machine-interface of the system (1).

3. System (1) according to claim 1 or 2, wherein
- the system (1) comprises at least one actuator (10) for providing feedback perceptible to the tongue and/or
- wherein the system (1), in particular the support sheet (2) and the sensing film (16), show a C-shape (33) with a central recess (34).

4. System (1) according to claim 3, wherein the system (1) comprises an electronic driving unit (11) configured to provide a drive signal to said at least one actuator (10),
- in particular for providing interactive feedback to a user of the system (1) and/or in response to a signal received from the sensor array (4),
- preferably wherein the system (1) comprises a, preferably wireless, communication module (12) for bidirectional communication with an external electronic device and/or for enabling the interactive feedback through interaction with an external electronic device.

5. System (1) according to one of the preceding claims, wherein an intraoral portion (13) of the system (1) features a maximum material thickness,
- in particular with respect to a stack (15) comprising the support sheet (2), a sensing film (16) bearing the sensor array (4) and a cover film (42),
of less than 2.0 mm, preferably of less than 1.5 mm, most preferably of less than 1.0 mm, and/or
- wherein the support sheet (2) is 3D-printed or thermoformed and/or
- wherein the support sheet (2) shows a thickness of less than 1.0 mm, preferably of less than 0.5 mm, most preferable of less than 0.3 mm.

6. System (1) according to one of the preceding claims, wherein the sensors (17) of the sensor array (4) are capacitive sensors (17),
- preferably wherein each of the capacitive sensors (17) is formed by a dielectric elastomer transducer (DET), comprising a soft and reversibly compressible dielectric layer sandwiched between two conformable electrodes,
- in particular wherein said at least one actuator (10) is formed by such a DET.

7. System (1) according to one of the preceding claims, wherein said support sheet (2) includes a lead (18) protruding from the oral cavity,
- preferably in the area of the incisors and/or
- with a width of less than the four upper incisors,
for supporting said at least one extraoral sensor (6), in particular said at least one interlabial sensor (7).

8. System (1) according to one of the preceding claims, wherein the sensor array (4) comprises
- at least one extraoral sensor (5) facing a buccal face (23) or labial face (24) of a tooth of the upper or lower jaw, preferably at the location of a canine teeth (25) and/or of at least one posterior teeth (21), preferably a pre-molar (22), and/or
- at least one intraoral sensor (5) facing a palatinal face (20) of a tooth (19) of the upper or lower jaw, preferably of a canine teeth (25) and/or a posterior teeth (21).

9. System (1) according to one of the preceding claims, wherein the sensor array (4) comprises one central sensor patch (26) located at the tongue resting position (27),
- preferably and two buccal sensor patches (28) facing left and right buccal side surfaces of teeth of the upper or lower jaw, preferably at the location of canine teeth and/or posterior teeth,
- most preferably and two palatinal sensor patches (29) facing left and right palatinal side surfaces of teeth of the upper or lower jaw, preferably of posterior teeth,
- in particular, wherein each of said sensor patches (26, 28, 29) features at least two, preferably at least three, separate sensors (17) for spatially and/or temporally resolving tongue pressure as exerted on the respective sensor patch (26, 28, 29).

10. System (1) according to one of the preceding claims, wherein said sensor array (4) is applied as a flexible sensing film (16) to a surface of said support sheet (2), for example by gluing or deepdrawing or thermoforming,
- in particular wherein said sensing film (16) has a thickness of less than 500 µm, preferably less than 100 µm, and most preferably less than 50 µm and/or is stretchable.

11. **Use of** an intraoral electronic tongue monitoring system (1), in particular according to one of the preceding claims, for measuring a pressure exerted by the tongue on single teeth of the upper or lower jaw, in particular on premolar and/or molar teeth, and/or
- for measuring a pressure distribution exerted by the tongue on a support sheet (2) of the system (1) and/or on a sensing film (16) of the system (1) and/or
- for measuring interlabial pressure and/or
- for detecting extraoral movements of the tongue, in particular for tongue training.

12. **Use of** an intraoral electronic tongue monitoring system (1), in particular according to one of the claims 1 to 10, as a human-machine-interface,
- in particular for controlling a software application, for example a video game, or an actuator through tongue movement, respectively,
- preferably wherein a tongue pressure distribution is spatially and/or temporally resolved by the system (1) and read out for controlling said software / said actuator,
- most preferably, wherein feedback perceptible to the tongue is provided to a user by at least one actuator (10) of the system (1) .

13. Use, in particular according to claim 11 or 12, of an intraoral electronic tongue monitoring system (1), in particular according to one of the claims 1 to 10,
- as a training device for training of tongue movement and position and/or tongue muscle tonus, in particular wherein said system (1) comprises at least one actuator (10) for providing feedback perceptible to the tongue,
or
- for treatment of snoring or sleep apnea or speech impediments.

14. **Process for fabricating** an intraoral electronic tongue monitoring system (1), in particular according to one of the claims 1 to 10, comprising a support sheet (2) to be attached to a human upper or lower jaw in an oral cavity (3) and a sensor array (4) arranged on the support sheet (2), **characterized in that**
- sensors (17) of the sensor array (4) are, at least partly, preferably fully, fabricated on a separate film,
- preferably wherein this film shows a thickness of less than 500 µm, most preferably of less than 100 µm,
to form a sensing film (16), and
- said sensing film (16) is attached to said support sheet (2),
- preferably wherein said film as well as the sensors (17) formed on the film are stretched during application of the sensing film (16) to the support sheet (2).

15. Process according to claim 14, wherein said support sheet (2) is fabricated
- by casting or 3d-printing a 3D-model (30) of an upper or lower jaw of an individual patient and by subsequent 3d-forming, in particular deepdrawing or thermo-forming, of the support sheet (2) using said 3D-model (30),
- preferably wherein said 3D-model (30) includes a foundation (31) for replicating a lead (18) designed for bearing extraoral, in particular interlabial, sensors (17) of the system (1),
or
- by acquiring 3D-data of a topology (32) of an upper or lower jaw of an individual patient,
- in particular using an intraoral optical 3D-scanner,
and by subsequent 3D-printing of the support sheet (2) as a negative of said topology (32), based on said 3D-data, such that the support sheet (2) accurately matches said topology (32).
